# EUROPEAN PATENT SPECIFICATION

(11) **EP 3 730 126 B1**
(45) Date of publication and mention of the grant of the patent: **14.05.2025**
(21) Application number: 18893272.7
(22) Date of filing: 18.12.2018
(51) Int. Cl.: A61K 9/08, A61K 47/12, A61K 47/18, A61K 47/26, A61K 38/17, A61K 9/00

(54) **LIQUID COMPOSITION COMPRISING VEGF ANTAGONIST**
VEGF-ANTAGONIST ENTHALTENDE FLÜSSIGE ZUSAMMENSETZUNG
COMPOSITION LIQUIDE COMPRENANT UN ANTAGONISTE DU VEGF

(30) Priority: 22.12.2017 KR 20170178693
(43) Date of publication of application: 28.10.2020
(73) Proprietor: Samsung Bioepis Co., Ltd., Incheon 21987 (KR)
(72) Inventor: KIM, Inae, Seoul 05090 (KR); JUNG, Youngseok, Incheon 22008 (KR); JANG, Yong Min, Seoul 05334 (KR); YOO, Wonjung, Busan 48093 (KR); LEE, Jaemin, Seoul 05536 (KR); KIM, Yongkook, Incheon 22003 (KR)
(74) Representative: Boult Wade Tennant LLP
(86) International application number: PCT/KR2018/016156
(87) International publication number: WO 2019/124946

(56) References cited:
- EP-A1- 3 790 532
- WO-A1-2014/160490
- WO-A1-2016/208989
- WO-A1-2017/117202
- WO-A2-2007/149334
- KR-A- 20080 015 780
- KR-A- 20090 018 807
- KR-A- 20170 000 356
- KR-B1- 101 482 304
- US-A1- 2013 101 584
- US-A1- 2017 080 086

## Description

### [TECHNICAL FIELD]

Provided is a stabilized liquid composition comprising: (1) 5 mg/ml to 100 mg/ml of aflibercept; (2) a buffer of 1 to 50 mM, comprising sodium phosphate and having pH ranging from 4 to 8; (3) a stabilizer of 7.8 to 10%(w/v), wherein the stabilizer is selected from the group consisting of trehalose and sucrose; and wherein the liquid composition does not comprise sodium chloride.

### [BACKGROUND ART]

Fusion protein drugs are more likely to cause physicochemical instability due to their larger molecular weight and complex structure than general protein drugs, and therefore the development of a suitable formulation is required. The chemical and physical stability of protein drugs can be optimized by various conditions such as pH condition, buffer selection, a concentration of protein, an excipient and temperature, etc. For development of a formulation which can ensure the stability of fusion proteins and maintain pharmacological activity of the fusion proteins, various means such as buffer modification, pH optimization and stabilizer addition, and the like have been used. Patent document WO 2016/208989 discloses stable liquid formulations comprising aflibercept.

EYLEA^{®}(aflibercept), a macular degeneration therapeutic agent, is a liquid formulation drug which has a composition of 40 mg/mL aflibercept, pH 6.2, 10 mM sodium phosphate, 40 mM NaCl, 5%(w/v) sucrose, and 0.03%(w/v) polysorbate 20. In the case of intraocular drugs, the regulatory standards for sub-visible particles are more stringent than those for other general biopharmaceuticals (see Table 1), and thus it is needed to develop a formulation considering the particulate aspect.

**[Table 1]**

| Sub-visible particle detection standards | | |
|---|---|---|
| Category | Number of particles | |

| | ≥10 um | ≥25 um |
|---|---|---|
| Small-volume (≤100 mL)injections | <6,000/container | <600/container |
| Large-volume (>100 mL)injections | <25/mL | <3/mL |
| Ophthalmic solution | <50/mL | <5/mL |

| | | |
|---|---|---|
| * Based on USP 788, 789, light obscuration test standards | | |

### [DISCLOSURE]

### [TECHNICAL PROBLEM]

Accordingly, the present disclosure provides a composition for stabilization, which enhances the stability of a protein drug, and a stable liquid composition of a protein drug comprising the same. Claimed is a liquid composition comprising: (1) 5 mg/ml to 100 mg/ml of aflibercept; (2) a buffer of 1 to 50 mM, comprising sodium phosphate and having pH ranging from 4 to 8; (3) a stabilizer of 7.8 to 10%(w/v), wherein the stabilizer is selected from the group consisting of trehalose and sucrose; and wherein the liquid composition does not comprise sodium chloride.

The liquid composition may further comprise a surfactant, for example, in an amount of 0.01 to 3 %(w/v) based on the total composition.

Another embodiment provides a pharmaceutical composition comprising the liquid composition.

For example, for the liquid composition or pharmaceutical composition, the protein may be one having a molecular weight of 10 to 500 kDa, 10 to 400 kDa, 10 to 300 kDa, 10 to 200 kDa, or 10 to 150 kDa. Claimed is that the protein is the VEGF antagonist aflibercept (having a molecular weight of about 97 to 115 kDa).

The pharmaceutical composition may be an ophthalmic composition, and particularly, may be parenteral formulation for intravitreal administration.

The liquid composition or pharmaceutical composition may be for intravitreal administration.

### [TECHNICAL SOLUTION]

Claimed is a liquid composition comprising: (1) 5 mg/ml to 100 mg/ml of aflibercept; (2) a buffer of 1 to 50 mM, comprising sodium phosphate and having pH ranging from 4 to 8; (3) a stabilizer of 7.8 to 10%(w/v), wherein the stabilizer is selected from the group consisting of trehalose and sucrose; and wherein the liquid composition does not comprise sodium chloride.

The protein content in the liquid composition may be 5 mg/ml to 100 mg/ml, 5 mg/ml to 80 mg/ml, 5 mg/ml to 60 mg/ml, 5 mg/ml to 50 mg/ml, 10 mg/ml to 100 mg/ml, 10 mg/ml to 80 mg/ml, 10 mg/ml to 60 mg/ml, 10 mg/ml to 50 mg/ml, 20 mg/ml to 100 mg/ml, 20 mg/ml to 80 mg/ml, 20 mg/ml to 60 mg/ml, 20 mg/ml to 50 mg/ml, 30 mg/ml to 100 mg/ml, 30 mg/ml to 80 mg/ml, 30 mg/ml to 60 mg/ml, or 30 mg/ml to 50 mg/ml, and for example, may be 5 mg/ml, 10 mg/ml, 20 mg/ml, 30 mg/ml, 40 mg/ml, 50 mg/ml, 60 mg/ml, 70 mg/ml, 80 mg/ml, 90 mg/ml, or 100 mg/ml.

The buffer may be one of pH 4 to 8, for example, pH 5.2 to 7.5, pH 5.2 to 7.2, pH 5.2 to 7, pH 5.2 to 6.8, pH 5.2 to 6.6, pH 5.2 to 6.4, pH 5.2 to 6.2, pH 5.5 to 7.5, pH 5.5 to 7.2, pH 5.5 to 7, pH 5.5 to 6.8, pH 5.5 to 6.6, pH 5.5 to 6.4, pH 5.5 to 6.2, pH 5.8 to 7.5, pH 5.8 to 7.2, pH 5.8 to 7, pH 5.8 to 6.8, pH 5.8 to 6.6, pH 5.8 to 6.4, pH 5.8 to 6.2, or pH 6.2.

According to the claims, the buffer comprises sodium phosphate.

The buffer may comprise one or more selected from the group consisting of phosphoric acid, acetic acid, citric acid, succinic acid, pharmaceutically acceptable salts (for example, sodium salt, potassium salt, etc.) of the acids, and histidine. In an embodiment, the buffer may comprise sodium phosphate, histidine, or a combination thereof. The buffer may be comprised at the concentration of about 1 mM to about 50 mM, about 1 mM to about 40 mM, about 1 mM to about 30 mM, about 1 mM to about 20 mM, about 1 mM to about 15 mM, about 1 mM to about 12 mM, about 5 mM to about 50 mM, about 5 mM to about 40 mM, about 5 mM to about 30 mM, about 5 mM to about 20 mM, about 5 mM to about 15 mM, about 5 mM to about 12 mM, about 8 mM to about 50 mM, about 8 mM to about 40 mM, about 8 mM to about 30 mM, about 8 mM to about 20 mM, about 8 mM to about 15 mM, about 8 mM to about 12 mM, or 10 mM, based on the total liquid composition.

According to the claims, the liquid composition does not comprise sodium chloride.

The stabilizer is selected from the group consisting of trehalose and sucrose.

According to the claims, the liquid composition comprises 7.8 to 10%(w/v) stabilizer.

In a specific embodiment, when the liquid composition comprises sodium phosphate as a buffer, and sucrose as a stabilizer, the liquid composition does not comprise sodium chloride.

The liquid composition may comprise the aforementioned contents of a protein, a buffer and a stabilizer, and a residual aqueous medium (for example, water (purified water), saline solution, injection water, etc.).

In one embodiment, the liquid composition may further comprise a surfactant, for example, in an amount of 0.001 to 3 %(w/v), 0.001 to 2 %(w/v), 0.001 to 1 %(w/v), 0.001 to 0.5 %(w/v), 0.001 to 0.1 %(w/v), 0.001 to 0.05 %(w/v), 0.01 to 3 %(w/v), 0.01 to 2 %(w/v), 0.01 to 1 %(w/v), 0.01 to 0.5 %(w/v), 0.01 to 0.1 %(w/v), 0.01 to 0.05 %(w/v), or 0.03 %(w/v), based on the total composition. The surfactant may be selected from any pharmaceutically acceptable surfactants which can disperse the protein evenly in the liquid composition medium. The surfactant may be a non-ionic surfactant; for example, at least one selected from the group consisting of polysorbates (for example, polysorbate 20 (polyoxyethylene (20) sorbitan monolaurate), polysorbate 40 (polyoxyethylene (20) sorbitan monopalmitate), polysorbate 60 (polyoxyethylene (20) sorbitan monostearate), polysorbate 80 (polyoxyethylene (20) sorbitan monooleate), wherein the numerical value behind the polyoxyethylene, i.e., (20), means the total number of oxyethylene groups (-(CH₂CH₂O)-); poloxamer (PEO-PPO-PEO copolymer; PEO: poly(ethylene oxide), PPO: poly(propylene oxide)), polyethylene-polypropylene glycol, polyoxyethylene compounds (for example, polyoxyethylene-stearate, polyoxyethylene alkyl ether (alkyl: C₁-C₃₀), polyoxyethylene monolauryl ether, alkylphenyl polyoxyethylene copolymer (alkyl: C₁-C₃₀), etc.), sodium dodecyl sulphate (SDS), and the like. For example, the surfactant may be polysorbates (for example, polysorbate 20).

The liquid composition provided in the present specification may be isotonic with a living tissue. For example, the osmotic pressure of the liquid composition may be about 200 mOsm/kg to about 400 mOsm/kg, for example about 250 mOsm/kg to about 300 mOsm/kg. Such an osmotic pressure may be adjusted by the stabilizer.

The electrical conductivity of the liquid composition provided in the present specification, may be about 0.1 mS/cm or more, for example, about 0.1 mS/cm to about 10 mS/cm, about 0.1 mS/cm to about 7 mS/cm, about 1 mS/cm to about 10 mS/cm, about 1 mS/cm to about 7 mS/cm, about 2.5 mS/cm to about 10 mS/cm, about 2.5 mS/cm to about 7 mS/cm, about 5 mS/cm to about 10 mS/cm, or about 5 mS/cm to about 7 mS/cm.

In the liquid composition provided in the present specification, the protein is the VEGF-specific fusion protein aflibercept having the following amino acid sequence of SEQ ID NO: 1.

Aflibercept amino acid sequence (SEQ ID NO: 1)

SDTGRPFVEM YSEIPEIIHM TEGRELVIPC RVTSPNITVT LKKFPLDTLI
PDGKRIIWDS RKGFIISNAT YKEIGLLTCE ATVNGHLYKT NYLTHRQTNT
IIDVVLSPSH GIELSVGEKL VLNCTARTEL NVGIDFNWEY PSSKHQHKKL
VNRDLKTQSG SEMKKFLSTL TIDGVTRSDQ GLYTCAASSG LMTKKNSTFV
RVHEKDKTHT CPPCPAPELL GGPSVFLFPP KPKDTLMISR TPEVTCVVVD
VSHEDPEVKF NWYVDGVEVH NAKTKPREEQ YNSTYRVVSV LTVLHQDWLN
GKEYKCKVSN KALPAPIEKT ISKAKGQPRE PQVYTLPPSR DELTKNQVSL
TCLVKGFYPS DIAVEWESNG QPENNYKTTP PVLDSDGSFF LYSKLTVDKS
RWQQGNVFSC SVMHEALHNH YTQKSLSLSP G

### (Disulfide bridge: 30-79; 124-185; 246-306; 352-410, Dimer: 211; 214)

The fusion protein may be recombinationally or synthetically produced.

The liquid composition provided in the present specification may be stably maintained at the high temperature of about 40°C for 4 weeks or more.

The term "stability is excellent" or "stably maintained" may mean that physical, chemical and/or biological properties and/or the structure of a protein in a composition can be maintained during storage (for example, during storage, a low protein polymer formation rate, a low protein aggregation rate, a low protein degradation rate, and/or a low denaturation rate, etc.). Various analysis techniques to measure the stability of the protein are well known in the related technical field.

For example, the liquid composition provided in the present specification, when the protein (antibody) content is 40 mg/ml, the change (%HMW at fourth week of storage - %HMW at week 0 (beginning of storage)) of the protein polymer formation rate or aggregation rate (High Molecular Weight %(w/v); %HMW) measured during storage at 40°C for 4 weeks with a conventional SEC (size exclusion chromatography) may be about less than 9, for example, about 8.8 or less or about 8.3 or less, but not limited thereto.

Another embodiment provides a pharmaceutical composition comprising the liquid composition. The pharmaceutical composition may further comprise pharmaceutically acceptable carriers, diluents, and/or excipients. The pharmaceutically acceptable carrier is that conventionally used, and may comprise one or more selected from the group consisting of lactose, dextrose, sucrose, sorbitol, mannitol, starch, acacia rubber, calcium phosphate, alginate, gelatin, calcium silicate, microcrystal cellulose, polyvinylpyrrolidone, cellulose, water (for example, purified water), saline solution, syrup, methyl cellulose, methylhydroxybenzoate, propylhydroxybenzoate, talc, magnesium stearic acid, mineral oil, and the like, but not limited thereto.

The liquid composition or pharmaceutical composition may be administered through an oral or parenteral route. In case of parenteral administration (for example, injection), it may be administered by intravenous administration, subcutaneous administration, intramuscular administration, intraperitoneal administration, endothelial administration, local administration, intranasal administration, intrapulmonary administration, intrarectal administration, intratumoral administration, intravitreal administration, etc.

In a specific embodiment, the liquid composition or pharmaceutical composition may be an ophthalmic solution comprising a VEGF antagonist as described above, and in this case, may be parenteral formulation to be administered into a vitreous humor of an eye.

Disclosed, but not claimed, is a method of stabilizing a protein or a method of preparing a stabilized liquid composition, comprising a step of mixing a protein with the aforementioned composition for stabilization.

Disclosed, but not claimed, is a method for stabilization of a protein or a method for preparation of a stabilized aqueous liquid composition, comprising a step of mixing
(1) a protein;
(2) a buffer comprising sodium phosphate, histidine, or a combination thereof;
(3) a stabilizer which does not comprise sodium chloride and/or comprises one or more selected from the group consisting of trehalose, sucrose and mannitol; and
(4) optionally, a surfactant

The specific description of kinds and contents of each component which is used for the method for stabilization of a protein or the method of preparation of a stabilized aqueous liquid composition is same as aforementioned.

### [ADVANTAGEOUS EFFECTS]

The present invention can inhibit production of polymers and/or aggregates and production of fragments and/or denaturation into charged variants which can be occurred during storage of the protein, thereby maintaining pharmacological effects of a protein in extended period of time, by providing a composition and liquid formulation for stabilization that allow a protein such as a fusion protein to stably maintain physical, chemical and/or biological efficacy for an extended period of time.

### [DESCRIPTION OF DRAWINGS]

FIG. 1 is a graph showing the %HMW (High-molecular weight %) change rate (Δ%HMW) for 4 weeks during 4 weeks storage at 40°C of test compositions measured in Example 2.
FIG. 2 is a graph showing the result of analyzing statistically significant factors using Minitab (Minitab, version 17), based on the Δ%HMW result for 4 weeks during 4 weeks storage at 40°C of test compositions measured in Example 2.
FIG. 3 is a graph showing the result of analyzing statistically significant factors using Minitab, based on the Δ%Acidic result for 4 weeks during 4 weeks storage at 40°C of test compositions measured in Example 3.
FIG. 4 is a graph showing the result of analyzing statistically significant factors using Minitab, based on the %RPA (Relative Potency Activity) change rate (Δ%RPA) result for 4 weeks during 4 weeks storage at 40°C of test compositions measured in Example 4.

### [MODE FOR INVENTION]

Hereinafter, the present invention will be described in more detail through examples and test examples. However, these examples and test examples are intended to illustrate the present invention, and should not be construed as limiting the present invention.

### Example 1. Preparation of liquid composition

Using a fusion protein functioning as a VEFG antagonist, aflibercept (a fusion protein of a VEGF binding site derived from extracellular domains of human VEGF receptors 1 and 2, and the Fc region of human IgG1; CAS Number: 862111-32-8; SEQ ID NO: 1) as a protein drug, liquid Compositions 1 to 14 of the protein drug were prepared as the composition of the following Table 2:

**[Table 2] (Compositions No. 4 and 6 are according to the claims)**

| Compos ition No. | Protein drug concentrat ion | pH | Buffer | Stabilizer | Surfactant |
|---|---|---|---|---|---|
| 1-3^{a} | 40 mg/mL | 6.2 | 10 mM sodium phosphate | 5%(w/v) Sucrose, 40 mM NaCl | 0.03%(w/v) PS20 |
| 4 | 40 mg/mL | 6.2 | 10 mM sodium phosphate | 8%(w/v) Sucrose | 0.03%(w/v) PS20 |
| 5 | 40 mg/mL | 6.2 | 10 mM sodium phosphate | 4.5%(w/v) Mannitol | 0.03%(w/v) PS20 |
| 6 | 40 mg/mL | 6.2 | 10 mM sodium phosphate | 8%(w/v) Trehalose | 0.03%(w/v) PS20 |
| 7 | 40 mg/mL | 6.2 | 10 mM sodium phosphate | 4.5%(w/v) dextrose | 0.03%(w/v) PS20 |
| 8 | 40 mg/mL | 6.2 | 10 mM histidine | 8%(w/v) Sucrose | 0.03%(w/v) PS20 |
| 9 | 40 mg/mL | 6.2 | 10 mM histidine | 4.5%(w/v) Mannitol | 0.03%(w/v) PS20 |
| 10 | 40 mg/mL | 6.2 | 10 mM histidine | 8%(w/v) Trehalose | 0.03%(w/v) PS20 |
| 11 | 40 mg/mL | 6.2 | 10 mM histidine | 4.5%(w/v) dextrose | 0.03%(w/v) PS20 |
| 12-14^{b} | 40 mg/mL | 6.2 | 10 mM sodium phosphate | 5%(w/v) Sucrose, 40 mM NaCl | 0.03%(w/v) PS20 |

| | | | | | |
|---|---|---|---|---|---|
| ^{a}Control (EYLEA^{®} composition-applied formulation), ^{b} Control (EYLEA^{®}) | | | | | |

### Example 2. Measurement of HMW content (%)

To test the stability of Compositions 1-14 prepared in Example 1, %HMW (% high molecular weight; based on weight), indicating an aggregation degree of the protein drug in the compositions was measured with SE-HPLC during storage for 4 weeks at the conditions of protein drug concentration 40 mg/mL, pH 6.2 and 40°C.

More specifically, a substance, which was detected earlier than the time when a monomer was detected, was defined as HMW, and %HMW was measured under the condition of 1.0 mL/min flow rate and 17 min injection time using HPLC (Waters 2695 separation module alliance) and column (Tosoh, TSK-gel G3000 SWXL) (%HMW = Area_{H}/Area_{TOTAL} x 100).

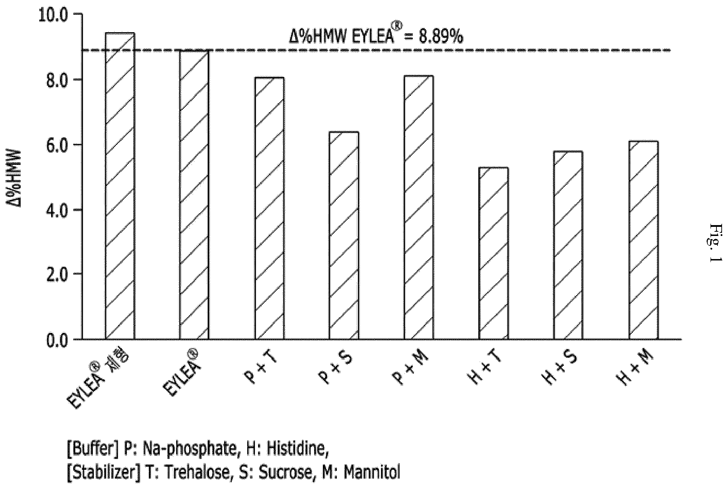

The measured %HMW (initial %HMW, 4^{th} week %HMW, Δ%HMW (4th week %HMW- initial %HMW) is showed in Table 3, and the 4^{th} week %HMW is shown in FIG. 1 (P: Na-phosphate, H: Histidine, T: Trehalose, S: Sucrose, M: Mannitol):

**[Table 3] (Compositions No. 4 and 6 are according to the claims)**

| Composition | Buffer | Stabilizer | Initial | 40°C, 4^{th} week | |
|---|---|---|---|---|---|
| No. | | | %HMW | %HMW | Δ%HMW |
| 1-3^{a} (control) | 10 mM Na-phosphate | 5%(w/v) Sucrose, 40 mM NaCl | 0.52 | 9.92 | 9.40 |
| | | | [N=3, SD:0.02] | [N=3, SD: 0.19] | [N=3, SD: 0.21] |
| 4 | | 8%(w/v) Sucrose | 0.52 | 6.92 | 6.40 |
| 5 | | 4.5%(w/v) Mannitol | 0.56 | 8.68 | 8.12 |
| 6 | | 8%(w/v) Trehalose | 0.55 | 8.62 | 8.07 |
| 7 | | 4.5%(w/v) Dextrose | 0.54 | 16.85 | 16.31 |
| 8 | 10 mM Histidine | 8%(w/v) Sucrose | 0.53 | 6.31 | 5.78 |
| 9 | | 4.5%(w/v) Mannitol | 0.54 | 6.64 | 6.10 |
| 10 | | 8%(w/v) Trehalose | 0.51 | 5.84 | 5.33 |
| 11 | | 4.5%(w/v) Dextrose | 0.53 | 13.19 | 12.66 |
| 12-14^{b} (control) | EYLEA^{®} | | 1.46 | 10.35 | 8.89 |
| | | | [N=3, SD:0.00] | [N=3, SD: 0.07] | [N=3, SD: 0.07] |

| | | | | | |
|---|---|---|---|---|---|
| ^{a}Control (EYLEA^{®} composition-applied formulation) ^{b} Control (EYLEA^{®}) | | | | | |

As shown in Table 3 and FIG. 1, in all the test compositions comprising sucrose, trehalose or mannitol as a stabilizer, it was shown that both %HMW at the 4^{th} week and the rate of increase of %HMW at the 4^{th} week (compared to %HMW at the initial (0 week)) were lower than those of the control (EYLEA^{®} and EYLEA^{®} composition-applied formulation).

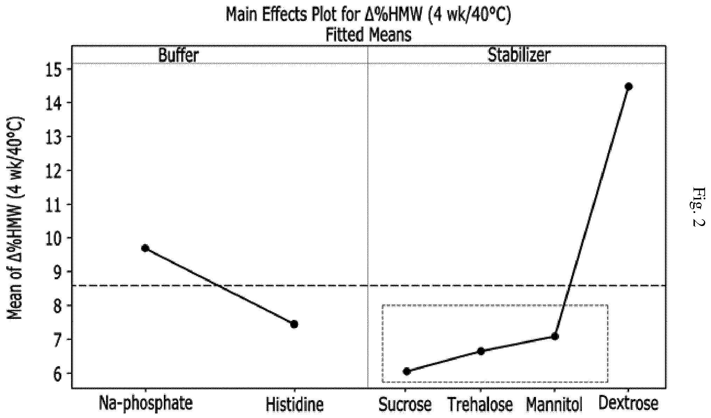

The result of analyzing statistically significant factors based on the Δ%HMW result shown in Table 3 is shown in FIG. 2. In FIG. 2, it was confirmed that the factors, which significantly affected Δ%HMW, were mannitol, sucrose, and trehalose, as a stabilizer. As shown in FIG. 2, there was no large difference depending on the kind of buffer, and in case of stabilizer, it was shown that %HMW was lower when using mannitol, sucrose or trehalose, compared to the composition using dextrose.

For the formulation comprising mannitol, sucrose, or trehalose as a stabilizer which was confirmed to lower Δ%HMW as above, the same test as above was repeatedly performed (n=3), and the obtained results are shown in Table 4:

**[Table 4] (Compositions No. 1-3, 4-6 are according to the claims)**

| Composition No. | Buffer | Stabilizer | Initial | 40°C, 4^{th} week | |
|---|---|---|---|---|---|
| | | | %HMW | %HMW | Δ%HMW |
| 1-3 | 10 mM Na-phosphate | 8% Trehalose | 0.78 | 8.56 | 7.78 |
| | | | [N=3, SD:0.01] | [N=3, SD:0.10] | [N=3, SD:0.09] |
| 4-6 | 10 mM Na-phosphate | 8% Sucrose | 0.80 | 8.07 | 7.27 |
| | | | [N=3, SD:0.01] | [N=3, SD:0.10] | [N=3, SD:0.09] |
| 7-9 | 10 mM Na-phosphate | 4.5% Mannitol | 0.78 | 9.58 | 8.80 |
| | | | [N=3, SD:0.01] | [N=3, SD:0.06] | [N=3, SD:0.06] |
| 10-12 | 10 mM Histidine | 8% Trehalose | 0.81 | 8.75 | 7.93 |
| | | | [N=3, SD:0.01] | [N=3, SD:0.15] | [N=3, SD:0.14] |
| 13-15 | 10 mM Histidine | 8% Sucrose | 0.78 | 8.41 | 7.63 |
| | | | [N=3, SD:0.00] | [N=3, SD:0.17] | [N=3, SD:0.17] |
| 16-18 | 10 mM Histidine | 4.5% Mannitol | 0.80 | 9.32 | 8.52 |
| | | | [N=3, SD:0.02] | [N=3, SD:0.12] | [N=3, SD:0.12] |
| 19-21 | 10 mM Na-phosphate | 5%(w/v) Sucrose, 40 mM NaCl | 0.82 | 11.21 | 10.40 |
| | | | [N=3, SD:0.00] | [N=3, SD:0.39] | [N=3, SD:0.39] |

As shown in Table 4, the formulation comprising mannitol, sucrose, or trehalose as a stabilizer showed lower Δ%HMW compared to the control, indicating that they have superior stability, and such superior stability was repeatedly achieved.

### Example 3. Measurement of Acidic content (%) and Main content (%)

Compositions 1-10 (Protein drug concentration: 40 mg/mL; pH 6.2; Surfactant: 0.03%(w/v) PS20) as shown in Table 5 were prepared using mannitol, sucrose, or trehalose as a stabilizer of which superior stabilizing effect was confirmed in Example 1 and Table 3, and using dextrose as a stabilizer for a control. To test the stability of these compositions, %Acidic (% Acidic variants content; based on weight) and %Main (content of a protein maintaining a surface charge of the initial condition in a charge variant aspect (based on weight)), which indicate the denaturation degree of the protein drug in the compositions, were measured using imaged capillary isoelectric focusing (icIEF) during storage for 4 weeks at 40°C.

More specifically, %Acidic, %Basic, and %Main were measured by analyzing acidic, main and basic isoforms of a sample, which was incubated after treating Enzyme (Sialidase A, Sigma-Aldrich), under the condition of 110 sec sample injection duration, 2000 psi sample injection pressure using icIEF instrument (Protein simple, iCE3).

The measured %Acidic and %Main (initial, 4^{th} week, 4^{th} week result - initial result (Δ%Acidic and Δ%Main)) are shown in Table 5:

**[Table 5] (Compositions No. 1 and 3 are according to the claims)**

| Com posit ion No. | Buffer | Stabilizer | icIEF | | | | | |
|---|---|---|---|---|---|---|---|---|
| | | | Initial | | 40°C, 4^{th} week | | | |
| | | | %Aci dic | %Mai n | %Acid ic | %Mai n | Δ%Aci dic | Δ%Ma in |
| 1 | 10 mM Na-phosphate | 8% Sucrose | 66.0 | 31.4 | 79.6 | 15.6 | 13.6 | -15.9 |
| 2 | | 4.5% Mannitol | 64.9 | 30.4 | 78.8 | 15.6 | 13.9 | -14.8 |
| 3 | | 8% Trehalose | 65.4 | 31.1 | 78.2 | 15.6 | 12.8 | -15.6 |
| 4 | | 4.5% Dextrose | 67.9 | 29.2 | N/A^{a} | | | |
| 5 | 10 mM Histidine | 5% Sucrose, 40 mM NaCl | 65.7 | 31.5 | 76.7 | 16.3 | 11.0 | -15.2 |
| 6 | | 8% Sucrose | 65.7 | 31.8 | 79.4 | 15.8 | 13.7 | -15.9 |
| 7 | | 4.5% Mannitol | 65.5 | 31.5 | 78.4 | 14.8 | 12.9 | -16.7 |
| 8 | | 8% Trehalose | 65.4 | 31.5 | 77.1 | 16.1 | 11.7 | -15.4 |
| 9 | | 135 mM NaCl | 65.9 | 31.2 | 78.1 | 15.8 | 12.2 | -15.4 |
| 10 | | 4.5% Dextrose | 66.7 | 28.9 | N/A^{a} | | | |

| | | | | | | | | |
|---|---|---|---|---|---|---|---|---|
| ^{a} N/A: Not available (Acidic, Main, and Basic peak were not distinguished) | | | | | | | | |

As shown in Table 5, all of the tested compositions showed lower change degrees in both %Acidic and %Main compared to the comparative composition comprising dextrose as the stabilizer, which indicates that the test compositions have increased stabilities.

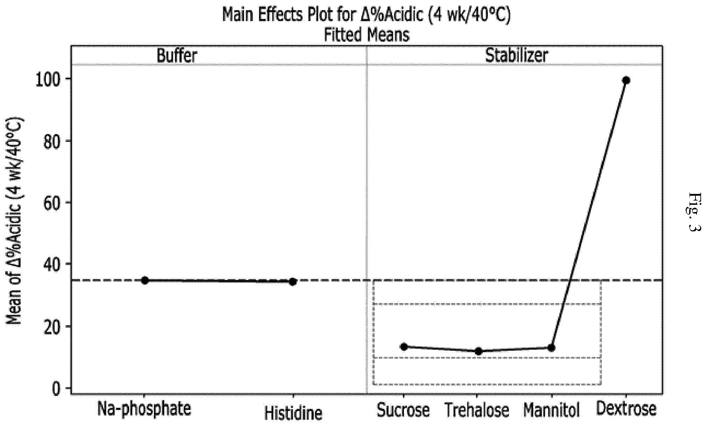

Statistically significant factors determined on the basis of the result of Table 5 were analyzed and the results are shown in FIG. 3. As shown in FIG. 3, it was confirmed that there was no significant difference of Δ%Acidic according to buffer kinds, but all the stabilizers except for dextrose significantly affected Δ%Acidic.

### Example 4. Measurement of VEGF binding efficacy of Aflibercept

To test the maintenance of activity of aflibercept which is a pharmacological active ingredient in Compositions 1-14 of Table 2 prepared in Example 1, %RBA (Relative Binding Activity) and %RPA (Relative Potency Activity) of aflibercept in the compositions to VEFG were measured during storage for 4 weeks at the condition of protein drug concentration 40 mg/mL, pH 6.2 and 40°C.

More specifically, %RBA was measured by the following method: after performing VEGF 165 coating (including washing and blocking; R&D system) on MaxiSorp 96 well plate (Nunc), Aflibercept and secondary antibody (Sigma) were loaded thereon in order, and then, TMB ELISA substance solution (Thermo Fisher Scientific) was treated; thereafter, %RBA (Relative Binding Activity) was measured using ELISA leader (SpectraMax).

The measured %RBA at 0 week and 4^{th} week at 40°C and %RBA change rate for 4 weeks (Δ%RBA) are shown in Table 6:

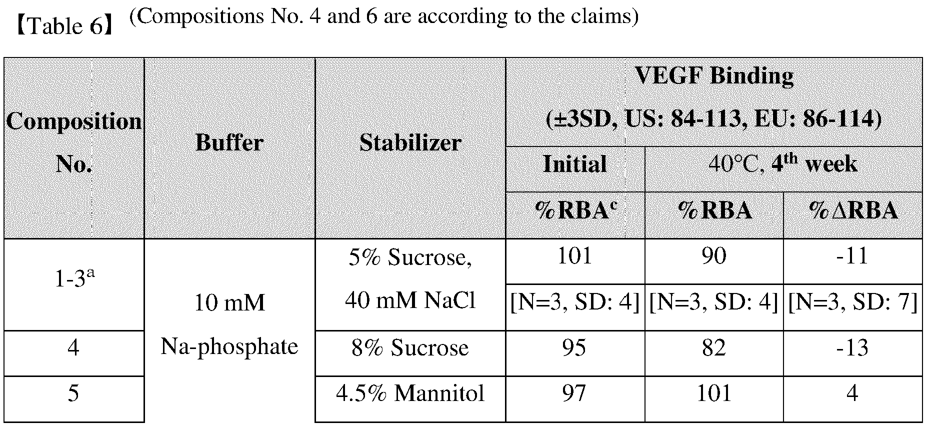

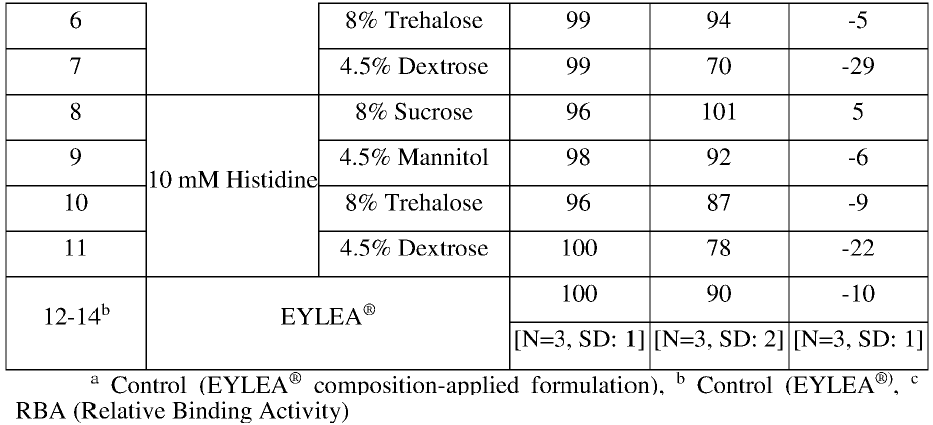

As shown in Table 6, it was shown that %RBA decrease rate was lower or equal level to that of the control in all the tested compositions, except for the composition comprising dextrose as the stabilizer, and such a result showed that aflibercept maintained the binding activity to VEGF well in all the tested compositions.

In addition, %RPA was measured by the following method: after loading and incubation of KDR 293 cell (Promega) in a 96 well plate in which aflibercept and VEGF were loaded in order, %RPA (Relative Potency Analysis) was analyzed using Envision microplate reader (Perkin Elmer, Envision 2014).

The measured %RPA at 0 week and 4th week at 40°C and %RPA change rate for 4 weeks (Δ%RPA) are shown in Table 7:

**[Table 7] (Compositions No. 4 and 6 are according to the claims)**

| Composition No. | Buffer | Stabilizer | VEGF Neutralization (±3SD, US: 83-118, EU: 92-111) | | |
|---|---|---|---|---|---|
| | | | Initial | 40°C, 4^{th} week | |
| | | | %RPA^{c} | %RPA | Δ%RPA |
| 1-3^{a} | 10 mM Na-phosphate | 5% Sucrose, 40 mM NaCl | 103 | 91 | -8 |
| | | | [N=3, SD:5] | [N=3, SD: 5] | [N=3, SD: 3] |
| 4 | | 8% Sucrose | 96 | 86 | -10 |
| 5 | | 4.5% Mannitol | 98 | 86 | -12 |
| 6 | | 8% Trehalose | 100 | 83 | -17 |
| 7 | | 4.5% Dextrose | 109 | 77 | -32 |
| 8 | 10 mM Histidine | 8% Sucrose | 102 | 97 | -5 |
| 9 | | 4.5% Mannitol | 97 | 97 | 0 |
| 10 | | 8% Trehalose | 98 | 95 | -3 |
| 11 | | 4.5% Dextrose | 109 | 89 | -20 |
| 12-14^{b} | EYLEA^{®} | | 105 | 95 | -11 |
| | | | [N=3, SD: 7] | [N=3, SD: 1] | [N=3, SD: 6] |

| | | | | | |
|---|---|---|---|---|---|
| ^{a} Control (EYLEA^{®} composition-applied formulation), ^{b} Control (EYLEA^{®}), ^{c} RPA (Relative Potency Activity) | | | | | |

As shown in Table 7, except for the composition comprising dextrose as the stabilizer, the decrease rate in %RPA of all the tested composition were lower or equal level to that of control, which indicates that the relative titre of aflibercept to VEGF neutralization capability can be maintained well in all the tested compositions.

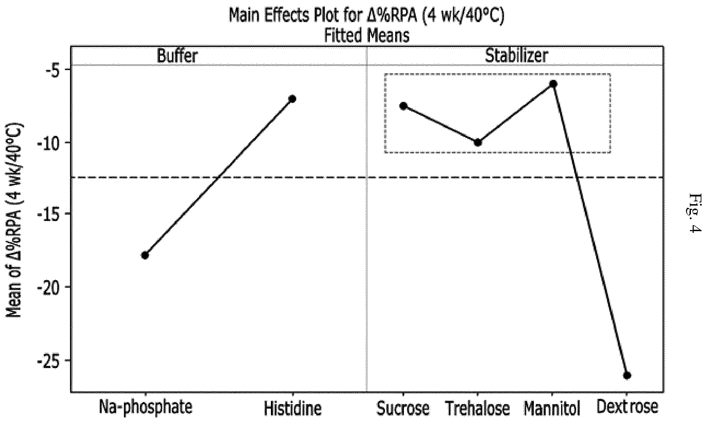

Statistically significant factors were analyzed based on the %RPA result in Table 7 and the obtained results are shown in FIG. 4. As shown in FIG. 4, all the tested buffers showed similar or improved %RPA decrease, compared to the control, and in case of stabilizer, all the tested stabilizers except for dextrose showed similar or less %RPA decrease compared to the control.

### Example 5. Measurement of stability of 6 kinds of compositions

%Main of 6 kinds of compositions (compositions 4, 5, 6, 8, 9 and 10 in Table 2) which were confirmed to have excellent stability in Examples 2-4, among compositions prepared in Example 1, was measured using CE-SDS (Capillary electrophoresis sodium dodecyl sulfate) (40°C, 4 weeks).

More specifically, %Main was measured by the following method: after thermal treatment at 70°C of a sample which is mixed with SDS sample buffer (AB Sciex) and BME (2-mercaptoethaol, Sigma Aldrich) (mixing ratio: about 1:50 (v:v)), %total protein area which was detected under 220 nm was analyzed, using CE analysis instrument (Beckman Coulter, PA800 plus) and 32 karat software (Beckman Coulter).

The measured %Main at 0 week and 4th week at 40°C and %Main change rate for 4 weeks (Δ%Main) are shown in Table 8:

**[Table 8] (Compositions No. 4 and 6 are according to the claims)**

| Comp osition No. | Buffer | Stabilizer | Initial | | 40°C, 4^{th} week | |
|---|---|---|---|---|---|---|
| | | | % Impurity | %Main | %Main | Δ%Main |
| 1-3^{a} | 10 mM Na-phosphate | 5%(w/v) Sucrose, 40 mM NaCl | 3.1 | 96.9 | 95.2 | -1.7 |
| | | | [N=3, SD:0.37] | [N=3, SD:0.37] | [N=3, SD:0.12] | [N=3, SD:0.24] |
| 4 | | 8%(w/v) Sucrose | 2.7 | 97.3 | 96.3 | -1.0 |
| 5 | | 4.5%(w/v) Mannitol | 3.1 | 96.9 | 95.9 | -1.0 |
| 6 | | 8%(w/v) Trehalose | 2.6 | 97.4 | 95.3 | -2.1 |
| 8 | 10 mM Histidine | 8%(w/v) Sucrose | 2.0 | 98.0 | 96.3 | -1.7 |
| 9 | | 4.5%(w/v) Mannitol | 2.9 | 97.0 | 96.6 | -0.4 |
| 10 | | 8%(w/v) Trehalose | 2.1 | 97.8 | 96.3 | -1.5 |

| | | | | | | |
|---|---|---|---|---|---|---|
| ^{a}Control (EYLEA^{®} composition-applied formulation) | | | | | | |

As shown in Table 8, the %Main decrease rate in tested 6 compositions were equivalent to or lower than that of the control. Such a result showed that all the tested 6 compositions possessed equivalent to or higher stability compared to the control.

## Claims

1. A liquid composition comprising:
(1) 5 mg/ml to 100 mg/ml of aflibercept;
(2) a buffer of 1 to 50 mM, comprising sodium phosphate and having pH ranging from 4 to 8;
(3) a stabilizer of 7.8 to 10%(w/v), wherein the stabilizer is selected from the group consisting of trehalose and sucrose; and
wherein the liquid composition does not comprise sodium chloride.

2. The liquid composition according to claim 1, wherein the amount of the aflibercept is 20 mg/ml to 60 mg/ml.

3. The liquid composition according to claim 1 or claim 2, wherein the pH of the liquid composition is 5.8 to 6.2.

4. The liquid composition according to any one of claims 1 to 3, wherein the concentration of the buffer in the composition is 1 to 20 mM.

5. The liquid composition according to any one of the preceding claims, wherein the amount of the stabilizer is 7.8 to 8.2%(w/v).

6. The liquid composition according to any one of the preceding claims, further comprising a surfactant in an amount of 0.01 to 0.1%(w/v).

7. The liquid composition according to claim 6, wherein the surfactant is at least one selected from the group consisting of polysorbate 20, polysorbate 80, and a combination thereof.

8. The liquid composition according to any one of the preceding claims, which is aqueous and isotonic.

9. The liquid composition according to any one of the preceding claims, wherein the composition is aqueous and has osmotic pressure of 200 to 400 mOsm/kg.

10. The liquid composition according to any one of the preceding claims, wherein the composition comprises:
(1) 30 mg/ml to 50 mg/ml of aflibercept;
(2) a buffer of 1 to 20mM, comprising sodium phosphate;
(3) a sucrose of 7.8 to 10%(w/v); and
(4) a polysorbate 20 of 0.01 to 0.1 %(w/v);
wherein the liquid composition has pH of 6.2 and does not comprise sodium chloride.

11. A parenteral formulation for intravitreal administration, comprising the liquid composition according to any one of the preceding claims.

## Patentansprüche

1. Flüssige Zusammensetzung, umfassend:
(1) 5 mg/ml bis 100 mg/ml Aflibercept;
(2) einen Puffer von 1 bis 50 mM, der Natriumphosphat enthält und einen pH-Wert im Bereich von 4 bis 8 aufweist;
(3) einen Stabilisator von 7,8 bis 10 % (Gew./Vol.), wobei der Stabilisator aus der Gruppe ausgewählt ist, die aus Trehalose und Saccharose besteht; und
wobei die flüssige Zusammensetzung kein Natriumchlorid enthält.

2. Flüssige Zusammensetzung nach Anspruch 1, bei der die Menge an Aflibercept 20 mg/ml bis 60 mg/ml beträgt.

3. Flüssige Zusammensetzung nach Anspruch 1 oder 2, bei der der pH-Wert der flüssigen Zusammensetzung 5,8 bis 6,2 beträgt.

4. Flüssige Zusammensetzung nach einem der Ansprüche 1 bis 3, bei der die Konzentration des Puffers in der Zusammensetzung 1 bis 20 mM beträgt.

5. Flüssige Zusammensetzung nach einem der vorhergehenden Ansprüche, bei der die Menge des Stabilisators 7,8 bis 8,2 % (Gew. /Vol.) beträgt.

6. Flüssige Zusammensetzung nach einem der vorhergehenden Ansprüche, die ferner ein Tensid in einer Menge von 0,01 bis 0,1 % (Gew./Vol.) enthält.

7. Flüssige Zusammensetzung nach Anspruch 6, bei der das Tensid mindestens eines ist, das aus der Gruppe ausgewählt ist, die aus Polysorbat 20, Polysorbat 80 und einer Kombination davon besteht.

8. Flüssige Zusammensetzung nach einem der vorhergehenden Ansprüche, die wässrig und isotonisch ist.

9. Flüssige Zusammensetzung nach einem der vorhergehenden Ansprüche, wobei die Zusammensetzung wässrig ist und einen osmotischen Druck von 200 bis 400 mOsm/kg aufweist.

10. Flüssige Zusammensetzung nach einem der vorhergehenden Ansprüche, wobei die Zusammensetzung umfasst:
(1) 30 mg/ml bis 50 mg/ml Aflibercept;
(2) einen Puffer von 1 bis 20 mM, der Natriumphosphat enthält;
(3) eine Saccharose von 7,8 bis 10 % (Gew./Vol.); und
(4) ein Polysorbat 20 von 0,01 bis 0,1 % (Gew./Vol.);
wobei die flüssige Zusammensetzung einen pH-Wert von 6,2 aufweist und kein Natriumchlorid enthält.

11. Parenterale Formulierung zur intravitrealen Verabreichung, umfassend die flüssige Zusammensetzung nach einem der vorhergehenden Ansprüche.

## Revendications

1. Composition liquide, comprenant :
(1) de 5 mg/ml à 100 mg/ml d'aflibercept ;
(2) un tampon de 1 à 50 mM, comprenant du phosphate de sodium et présentant un pH dans la plage de 4 à 8 ;
(3) de 7,8 à 10 % (p/v) de stabilisant, dans laquelle le stabilisant est choisi dans le groupe constitué de tréhalose et de saccharose ; et
dans laquelle la composition liquide ne comprend pas de chlorure de sodium.

2. Composition liquide selon la revendication 1, dans laquelle la quantité d'aflibercept est de 20 mg/ml à 60 mg/ml.

3. Composition liquide selon la revendication 1 ou la revendication 2, dans laquelle le pH de la composition liquide est de 5,8 à 6,2.

4. Composition liquide selon l'une quelconque des revendications 1 à 3, dans laquelle la concentration du tampon dans la composition est de 1 à 20 mM.

5. Composition liquide selon l'une quelconque des revendications précédentes, dans laquelle la quantité du stabilisant est de 7,8 à 8,2 % (p/v).

6. Composition liquide selon l'une quelconque des revendications précédentes, comprenant en outre un tensioactif en une quantité de 0,01 à 0,1 %(p/v).

7. Composition liquide selon la revendication 6, dans laquelle le tensioactif est au moins un tensioactif choisi dans le groupe constitué du polysorbate 20, du polysorbate 80 et d'une combinaison de ceux-ci.

8. Composition liquide selon l'une quelconque des revendications précédentes, qui est aqueuse et isotonique.

9. Composition liquide selon l'une quelconque des revendications précédentes, dans laquelle la composition est aqueuse et présente une pression osmotique de 200 à 400 mOsm/kg.

10. Composition liquide selon l'une quelconque des revendications précédentes, dans laquelle la composition comprend :
(1) de 30 mg/ml à 50 mg/ml d'aflibercept ;
(2) un tampon de 1 à 20 mM, comprenant du phosphate de sodium ;
(3) de 7,8 à 10 % (p/v) de saccharose ; et
(4) de 0,01 à 0,1 % (p/v) de polysorbate 20 ;
dans laquelle la composition liquide présente un pH de 6,2 et ne comprend pas de chlorure de sodium.

11. Formulation parentérale pour administration intravitréenne, comprenant la composition liquide selon l'une quelconque des revendications précédentes.
